# FASCICULE DE BREVET EUROPEEN

(11) **EP 0 804 539 B1**
(45) Date de publication et mention de la délivrance du brevet: **19.01.2000**
(21) Numéro de dépôt: 94925522.8
(22) Date de dépôt: 19.08.1994
(51) Int. Cl.: C12G 3/08

(54) **PROCEDE ET DISPOSITIF AUTOMATIQUE DE STABILISATION TARTRIQUE DES VINS**
VERFAHREN UND VORRICHTUNG ZUR WEINSTEINSTABILISIERUNG VON WEIN
AUTOMATIC METHOD AND DEVICE FOR TARTARIC STABILISATION OF WINES

(30) Priorité: 27.08.1993 FR 9310328
(43) Date de publication de la demande: 05.11.1997
(73) Titulaire: INSTITUT NATIONAL DE LA RECHERCHE AGRONOMIQUE (INRA), 75341 Paris Cédéx 07 (FR); EURODIA INDUSTRIE SA, 91320 WISSOUS (FR)
(72) Inventeur: ESCUDIER, Jean-Louis, F-11110 Armissan (FR); SAINT-PIERRE, Bernard, F-43110 Frontignan (FR); BATLLE, Jean-Louis, F-11100 Narbonne (FR); MOUTOUNET, Michel, F-34080 Montpellier (FR)
(74) Mandataire: Michelet, Alain
(86) Numéro de dépôt international: FR9401018
(87) Numéro de publication internationale: WO9506110

(56) Documents cités:
- FR-A- 2 574 939
- US-B- 572 726
- DIE WEINWIRTSCHAFT, vol.114, no.1/2, 11 Janvier 1978, DE pages 28 - 33 W. POSTEL & E. PRASCH 'Untersuchungen zur Weinsteinstabilisierung von Wein durch Elektrolyse.'

## Description

L'invention concerne un procédé et un dispositif automatique de stabilisation tartrique des vins.

On sait que l'acide tartrique est un constituant caractéristique du raisin. Ces sels sont relativement peu solubles. En raison de la teneur en ions tartrates, potassium et calcium du vin, l'hydrogénotartrate de potassium (ou bitartrate de potassium) et le tartrate de calcium tendent naturellement à précipiter dans les vins.

Le développement de la cristallisation dépend de divers facteurs, notamment de la température, du pH, de la teneur an éthanol et de la présence de constituants inhibant la formation de nucleï ou empêchant le grossissement des cristaux.

Considérant la richesse du raisin en acide tartrique, potassium et calcium, ainsi que les phénomènes qui régissent les équilibres ioniques, les vins bruts sont naturellement en état de sursaturation pour les sels de potassium et de calcium de l'acide tartrique. Une forte proportion d'hydrogénotartrate de potassium se dépose sous forme cristalline, pendant et après la fermentation alcoolique.

Ces cristallisations apparaissent en fonction des conditions de conservation des vins et de l'évolution de leur état colloïdal. Elles sont généralement lentes et aléatoires, compte tenu de la complexité des paramètres induisant ou retardant la cristallisation.

Lorsque les précipitations des cristaux ont lieu en bouteilles, elles sont considérées comme un défaut nuisant à la présentation des vins et à l'image de marque du produit mis sur le marché.

Compte tenu des exigences d'un marché de plus an plus concurrentiel, il est demandé aux producteurs de vins de fournir des produits stables dans le temps au niveau tartrique.

C'est pourquoi de nombreuses recherches ont été conduites pour caractériser l'état de sursaturation du vin, mettre au point des tests de mesure de la stabilité et développer des procédés industriels de stabilisation vis-à-vis des précipitations tartriques.

Les techniques industriellement utilisées pour prévenir les risques de précipitation en bouteilles ont pour objet, soit de maintenir l'état de sursaturation, soit d'induire une précipitation par abaissement de la température du vin.

Les techniques du premier type procèdent par addition d'acide métartrique. Elles sont destinées à des vins jeunes, à court circuit de distribution.

Les autres techniques, qui font appel à l'utilisation de la réfrigération, se sont bien développées. Elles sont les plus largement employées et sont d'ailleurs les seules autorisées dans les pays membres de la CEE. Ces techniques sont efficaces principalement sur les vins blancs. Sur les vins rouges, en dépit des améliorations apportées aux matériels utilisés et du développement des méthodes de contrôle, la prévention de la précipitation des cristaux en bouteilles n'est pas définitivement acquise.

De plus, certains oenologues estiment que les qualités organoleptiques des vins peuvent être affectées par un traitement de stabilisation au froid. Ces constatations sont attribuables d'une part, aux techniques de filtration qu'il est nécessaire de mettre en oeuvre avant la réfrigération pour faciliter les phénomènes de cristallisation et d'autre part, aux filtrations effectuées pour éliminer les cristaux et clarifier le vin à froid.

Les travaux déjà effectués ont également montré la possibilité de prévenir les risques de précipitations de tartre par d'autres procédés tels que l'osmose inverse, l'échange d'ions ou encore l'électrodialyse.

L'examen de ces différentes techniques montre que lélectrodialyse et l'échange d'ions sont les deux seules à ce jour à assurer une garantie totale d'efficacité de traitement.

La technique de l'échange d'ions sur résines de type cationique conduit à un apport d'ions exogènes par échange avec ceux que l'on extrait du vin alors que l'électrodialyse est une technique soustractive d'ions, anions et cations, au travers de membranes sélectives, alternativement cationiques et anioniques.

Ainsi, le principe de l'échange d'ions de type cationique induit potentiellement des modifications de la constitution du vin qui sont plus importantes que lorsque la technique d'électrodialyse est utilisée.

De plus, la régénération des résines échangeuses d'ions utilisées, entraîne des rejets polluants.

L'application de l'électrodialyse à la stabilisation tartrique des vins est connue depuis plusieurs années. Elle permet d'extraire sélectivement les anions et les cations du vin en quantité maîtrisable et déterminée par avance. L'électrolyse n'est cependant pas encore utilisée à un niveau industriel.

Le document FR-A-2 574 939 décrit un test de laboratoire, permettant de contrôler l'efficacité d'un procédé de traitement du vin, ce traitement se traduisant par une variation de concentration d'un type d'ions déterminé. Ce contrôle consiste à mesurer la conductivité du vin avant et après traitement, puis à calculer notamment la température de saturation, pour déterminer si le vin est stable ou non.

Le document Die Weinwirtschaft (vol. 114, n° 1/2, 1978, pages 28-33) décrit un traitement du vin par électrodialyse. En fonction de la teneur initiale du vin en potassium, ou de sa conductivité initiale, on adopte un taux de traitement par électrodialyse, traduite en élimination de potassium et en diminution de la conductivité. Ce traitement est basé sur l'élimination de 100 à 350 mg/l de potassium, jugée nécessaire pour obtenir un vin stable.

Cependant, des travaux ont montré qu'il n'existe pas de relation entre la teneur initiale en potassium d'un vin (ou sa conductivité initiale) et le taux de traitement par électrodialyse nécessaire pour obtenir un vin stable.

Ainsi, l'invention a pour objet un procédé de stabilisation tartrique des vins, mettant en oeuvre un traitement par électrodialyse, qui garantit la qualité et l'efficacité du traitement.

L'invention concerne un procédé régulé tartrique des vins comprenant les étapes suivantes:
- prélèvement d'un échantillon du vin à stabiliser;
- détermination du degré d'instabilité spécifique dudit vin, par une mesure prédictive de conductivité correspondant à celle qu'aurait le vin s'il était stable;
- si le vin est instable, traitement dudit vin à stabiliser selon une technique membranaire d'électrodialyse jusqu'à l'obtention de son état de stabilité.

Selon une caractéristique préférée de l'invention, la mesure prédictive de conductivité est transformée en une valeur de consigne correspondant à la chute de conductivité nécessaire à la stabilisation du vin à traiter, le vin à stabiliser étant soumis en continu à un traitement par électrodialyse jusqu'à ce que la valeur de consigne soit atteinte.

De façon préférée, la mesure prédictive de conductivité est obtenue à partir d'un calcul de modélisation.

Le procédé selon l'invention présente également les caractéristiques suivantes, prises isolément ou en combinaison:
- le traitement est effectué sur la totalité du vin à stabiliser, de façon continue, en un seul cycle;
- le traitement est effectué, de façon continue, par cycles successifs, un volume intermédiaire étant traité à chaque cycle, jusqu'au traitement de la totalité du vin à stabiliser;
- le vin à stabiliser est filtré avant le traitement par électrodialyse;
- le traitement est limité à un taux maximum de désionisation, préalablement déterminé, par exemple d'environ 30%;
- la concentration en ions du concentré résultant du traitement par électrodialyse est maintenue sous un seuil déterminé;
- le maintien sous ledit seuil est assuré par la régulation de la conductivité du concentré par apport d'eau.

L'invention a également pour objet un dispositif automatique de stabilisation tartrique des vins.

Ainsi, l'invention concerne un dispositif automatique de stabilisation tartrique des vins comprenant:
- un appareil de test pour déterminer le degré d'instabilité spécifique du vin à stabiliser, par une mesure prédictive de conductivité correspondant à la conductivité qu'aurait le vin s'il était stable;
- un dispositif de traitement comprenant un électrodialyseur et recevant le vin à stabiliser;
- un système de contrôle-commande pour gérer automatiquement le dispositif de traitement, en fonction du degré d'instabilité du vin à traiter, déterminé par l'appareil de test.

De façon préférée, la mesure prédictive de conductivité est transformée en une valeur de consigne correspondant à la chute de conductivité nécessaire à la stabilisation du vin à traiter.

Le dispositif de stabilisation selon l'invention présente également les caractéristiques suivantes prises isolément ou en combinaison:
- le dispositif de traitement comprend en outre un dispositif de mesure pour mesurer la conductivité du vin en sortie de l'électrodialyseur, le système de contrôle assurant le retour en continu du vin à stabiliser dans l'électrodialyseur par des moyens appropriés, si la valeur de consigne n'est pas atteinte;
- le système de contrôle-commande assure l'évacuation du vin du dispositif de traitement grâce à des moyens appropriés, lorsque la valeur de consigne est atteinte;
- le dispositif de stabilisation est utilisé entre une première cuve comprenant la totalité du vin à stabiliser et une deuxième cuve qui recueille le vin stabilisé;
- le dispositif de traitement est directement relié à ladite première cuve comprenant la totalité du vin à stabiliser;
- le dispositif de traitement comprend une cuve intermédiaire associée à l'électrodialyseur et recevant un volume de vin à stabiliser;
- lorsque le volume de vin présent dans la cuve intermédiaire au début du traitement a été évacué, le système de contrôle agit sur des moyens appropriés pour assurer son remplissage par un nouveau volume de vin à stabiliser,
- la vidange et le remplissage de la cuve intermédiaire s'effectuent de telle sorte que le vin est traité de façon continue dans l'électrodialyseur,
- le dispositif de traitement comprend également un filtre placé en amont de l'électrodialyseur,
- le dispositif de traitement inclut en outre un circuit comprenant une boucle reliant l'électrodialyseur à une cuve recevant le concentré d'ions,
- le dispositif de traitement comprend des moyens pour réguler le seuil de conductivité dudit concentré,
- l'appareil de test réalise une fiche d'identité du vin,
- le dispositif de stabilisation comprend un dispositif de sécurité pour interdire le traitement du vin dans l'électrodialyseur lorsque les caractéristiques de celui-ci ne sont pas conformes à sa fiche d'identité.

L'invention sera mieux comprise et d'autres buts, avantages et caractéristiques de celle-ci apparaîtront plus clairement à la lecture de la description qui suit, faite au regard des dessins annexés, sur lesquels :
- la Figure 1 est une représentation schématique d'un dispositif de stabilisation tartrique des vins,
- la Figure 2 est une représentation détaillée d'un mode de réalisation du dispositif de traitement selon la Figure 1, et
- la Figure 3 illustre une cellule d'électrodialyse.

Les éléments communs aux différentes figures seront désignés par les mêmes références.

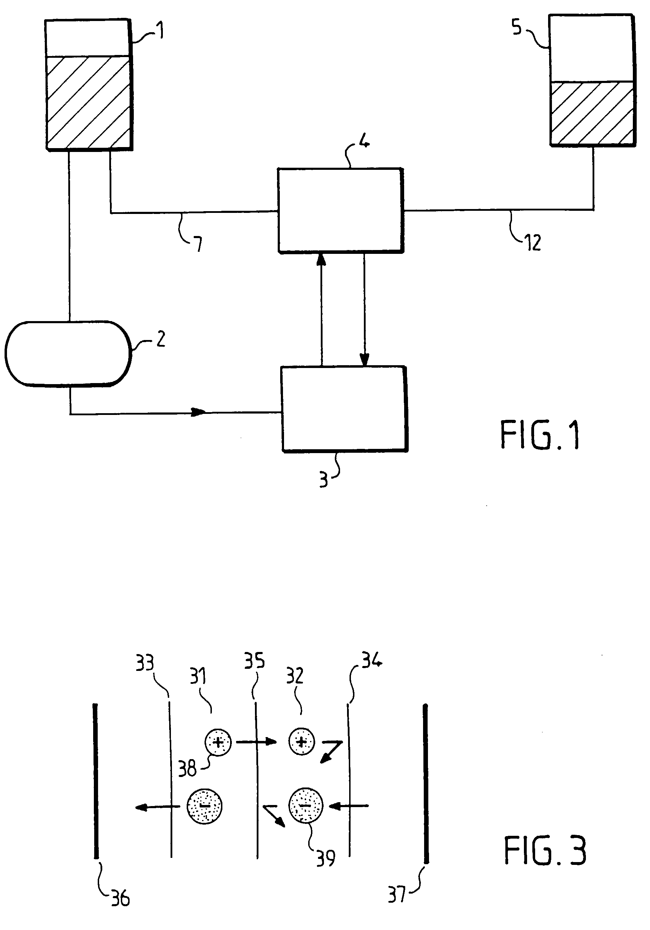

En référence à la Figure 1, le dispositif selon l'invention est utilisé entre une cuve 1 qui est remplie avec le vin à traiter et une cuve 5 qui reçoit le vin stabilisé. Ces deux cuves peuvent notamment être en place dans l'exploitation où le procédé est mis en oeuvre.

La référence 2 désigne un appareil de test. Cet appareil permet de déterminer le degré d'instabilité tartrique spécifique du vin à traiter, à partir d'un échantillon prélevé dans la cuve 1.

Comme on le verra plus loin, le résultat fourni par cet appareil sera utilisé au cours de la mise en oeuvre du procédé de traitement ultérieur.

Cet appareil 2 met en oeuvre un procédé comprenant les étapes suivantes :
- mise en oeuvre d'un test de stabilité cryogénique à partir d'un appareillage du type de ceux connus dans l'état de la technique (STABISAT) et adapté spécifiquement à l'étape suivante :
- exécution d'un calcul de modélisation, propre au vin à traiter, pour calculer précisément la valeur de la conductivité du vin une fois atteint son état de stabilité et,
- transformation de la valeur de conductivité obtenue en une valeur de consigne pour adapter cette valeur de conductivité au reste du procédé de stabilisation, lequel met en oeuvre la technique d'électrodialyse. La valeur de consigne de conductivité correspond au taux de désionisation ou à la chute de conductivité nécessaire à la stabilisation du vin à traiter.

Ainsi, l'appareil de test 2 met en oeuvre un procédé qui est basé sur l'analyse de la variation, pendant un temps suffisamment long, de la conductivité d'un échantillon placé à une température basse contrôlée et dans des conditions d'ensemencement au bitartrate de potassium de qualité sélectionnée, sous agitation constante.

Le procédé permet une modélisation du traitement au froid par contact (ajout de cristaux d'ensemencement et agitation). Cette modélisation a pour but de raccourcir le temps nécessaire à la mise en oeuvre de ce test. Différents modèles mathématiques peuvent être proposés en fonction des températures retenues (entre -4°C et 0°C) et de l'ajout éventuel d'éthanol pour accélérer la précipitation.

On constate que, par rapport aux tests de stabilité classique, le test mis en oeuvre par l'appareil 2 est plus précis et permet de caractériser l'état de stabilité du vin par une mesure de conductivité prédictive qui correspond à celle qu'aurait le vin s'il était stable.

Il convient de souligner que les deux premières étapes du procédé mis en oeuvre par l'appareil de test 2 sont indépendantes du procédé de traitement ultérieur. C'est la dernière étape du procédé qui permet de l'adapter à la technique utilisée lors du procédé de traitement proprement dit.

L'appareil de test 2 permet également l'identification et la reconnaissance du vin par sa conductivité ainsi que par un certain nombre de critères analytiques classiques en oenologie (par exemple : pH, turbidité, degré alcoolique etc...) avant que le procédé de traitement ne soit mis en oeuvre. Ces derniers critères sont déterminés par des dispositifs en communication avec l'appareil de test 2.

Ces différentes valeurs sont regroupées et constituent la fiche d'identité ou signature du vin. Leur intérêt sera décrit ultérieurement. Il convient déjà de préciser que la fiche d'identité n'est pas indispensable pour l'exécution du procédé de traitement.

Le résultat du test (valeur de consigne) ainsi que la fiche d'identité du vin sont transmis à un système de contrôle-commande 3 qui les mémorise.

L'analyse de l'échantillon peut être effectuée par l'appareil de test 2 dans l'exploitation où le procédé de stabilisation est mis en oeuvre. Elle peut également, dans certaines conditions, être effectuée en un lieu différent, par exemple dans un laboratoire. Dans ce cas, la transmission au système de contrôle-commande 3 du résultat du test et de la fiche d'identification peut être effectuée par des moyens classiques, tels que disquettes...

En fonction du degré d'instabilité du vin, c'est-à-dire de la valeur du taux de désionisation, le système 3 détermine si le vin est stable ou non.

Si le vin est stable, le procédé de traitement n'est pas mis en oeuvre sur la cuve 1.

Si au contraire, le vin est instable, le système de contrôle 3 autorise le démarrage du procédé de traitement par électrodialyse selon l'invention.

Le procédé de traitement est exécuté pour obtenir le taux de désionisation déterminé par le système de contrôle 3. Ce taux de désionisation correspond à la chute de conductivité nécessaire pour obtenir la stabilité. Elle est exprimée en % par rapport à la conductivité initiale mesurée par l'appareil de test 2.

Le procédé de traitement est donc basé sur une mesure de conductivité. L'expérience a en effet montré que la conductivité est linéairement corrélée à la teneur en potassium. Il est de plus aisé de réguler le traitement par électrodialyse au moyen d'une valeur de conductivité.

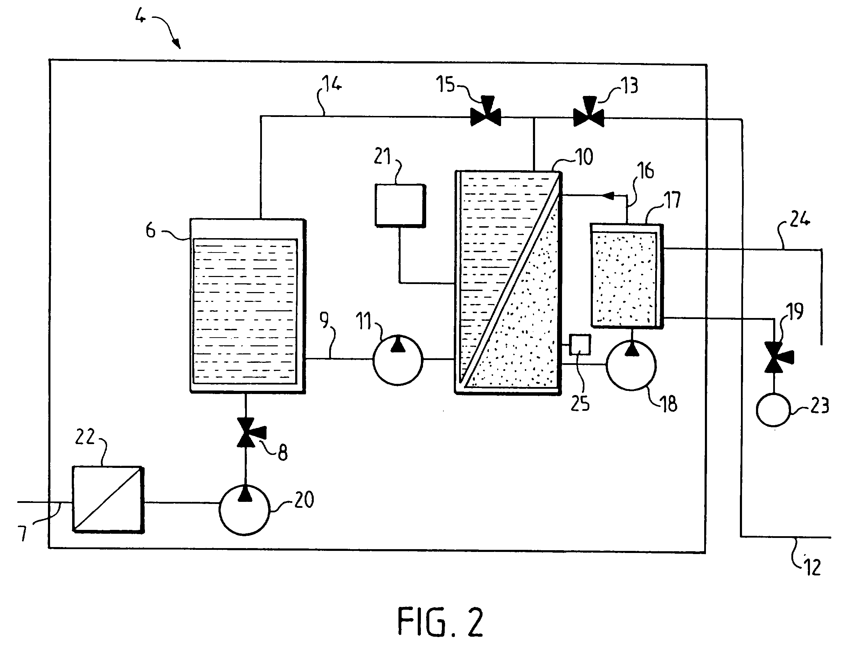

On se référera plus particulièrement à la figure 2 qui illustre plus en détail le dispositif de traitement 4.

Il convient tout d'abord de noter que dans le mode de réalisation de la figure 2, le traitement de l'ensemble de la cuve 1 contenant du vin estimé instable est effectué par cycle. A chaque cycle, un volume de vin de la cuve 1 est envoyé dans la cuve intermédiaire ou de travail 6 pour y être traité par l'électrodialyseur 10.

Ceci est particulièrement intéressant lorsque le volume de vin à traiter est important.

Cependant, on peut aussi envisager d'effectuer le traitement sur la totalité du vin à traiter, en un seul cycle. Dans ce cas, la cuve intermédiaire 6 n'est pas nécessaire et l'electrodialyseur 10 est directement relié à la cuve 1.

Le volume de vin à traiter est transmis de la cuve 1 à la cuve 6 par le conduit 7 et par des moyens appropriés constitués par une électrovanne 8. La pompe 20 puise le vin à traiter depuis la cuve 1 au travers d'un filtre 22. Le système de contrôle-commande 3 gère le remplissage de la cuve 6. Le vin contenu dans la cuve 6 est ensuite transmis par le conduit 9 à l'électrodialyseur 10 par l'intermédiaire d'une pompe 11. Dans l'électrodialyseur 10, le vin subit un traitement qui sera décrit plus loin. Ce traitement permet d'extraire du vin des ions potassium et tartrates et abaisse ainsi la conductivité du vin. Le circuit se referme vers la cuve 6 par l'électrovanne 15 et le circuit 14.

La conductivité est mesurée en continu dans l'électrodialyseur 10, grâce au dispositif de mesure 21.

Dès que l'abaissement de la conductivité assurant la stabilité du vin traité a été obtenu dans la boucle, le vin contenu dans la cuve 6 est envoyé via l'électrodialyseur 10, le conduit 9, la pompe 11, l'électrovanne 13 et le conduit 12, vers la cuve 5, alors que le retour par le conduit 14 est fermé par l'électrovanne 15. Le système de contrôle-commande 3 gère le procédé jusqu'au vidage complet de la cuve de travail 6.

Après que le vin à stabiliser contenu dans la cuve 6 a été transmis à la cuve 5 renfermant le vin stable, le système de contrôle-commande 3 commande de nouveau le remplissage de la cuve 6. Le traitement par électrodialyse peut alors être effectué sur un nouveau volume de vin à stabiliser.

Ces différents cycles sont effectués jusqu'à ce que la cuve 1 soit vide. Le traitement du vin est réalisé de façon continue, au niveau de l'électrodialyseur 10.

Il convient de noter que de façon préférée, le dispositif de stabilisation selon l'invention comporte un dispositif de sécurité qui verrouille le traitement par électrodialyse dans certaines conditions.

Comme on l'a vu précédemment, l'appareil de test 2 réalise une fiche d'identification du vin à traiter, sur la base d'un échantillon prélevé dans la cuve 1.

Si le vin est instable, il est conduit dans l'électrodialyseur 10. Au niveau de ce dernier, sont prévus un dispositif de mesure de conductivité 25 ainsi que des capteurs qui mesurent les mêmes critères analytiques que ceux qui figurent sur la fiche d'identification. Ces capteurs ne sont pas représentés sur la figure 2.

Les résultats de ces mesures sont transmis au système de contrôle-commande 3 qui les compare à la fiche d'identification du vin.

Si les critères analytiques en cause n'ont pas la même valeur, le système de contrôle-commande 3 interdit le démarrage du procédé de traitement par électrodialyse et le vin est évacué du dispositif.

Comme cela a été précisé plus haut, ce dispositif de sécurité n'est pas indispensable pour la mise en oeuvre du procédé de stabilisation selon l'invention. Il a essentiellement pour objet de vérifier que le vin transmis dans l'électrodialyseur 10 est bien identique au vin dont un échantillon a été analysé par l'appareil de test 2. Il permet ainsi d'éviter toute fraude lors du traitement des vins.

Le dispositif de traitement 4 comprend également un circuit pour le concentré (effluent) généré par l'électrodialyseur 10, comme cela sera expliqué plus loin. Ce circuit comprend une boucle 16 qui relie les membranes de l'électrodialyseur 10 à une cuve 17 contenant le concentré d'ions. Le concentré circule dans cette boucle par l'intermédiaire d'une pompe de circulation 18, située en amont des membranes.

Le concentré comprend essentiellement de l'eau et KCl au début du traitement, puis il s'enrichit en K, Na, Ca et tartrates au cours du traitement. La concentration augmente progressivement au cours du traitement. Elle est maintenue sous un seuil déterminé par régulation de la conductivité. La conductivité du concentré est mesurée par le dispositif 25. Le système de contrôle-commande 3 régule la conductivité grâce à des apports d'eau, qui sont effectués par l'électrovanne 19 en communication avec une distribution d'eau 23.

Le pH du concentré peut être utilement régulé et maintenu au pH du vin traité ou à un pH inférieur par un dispositif doseur-régulateur, afin d'éviter les précipitations des sels extraits sur les membranes de l'électrodialyseur 10 et de limiter le volume de rejet.

Ce dispositif doseur-régulateur n'est pas représenté sur la figure 2.

Le concentré est récupérable par débordement, à une concentration haute et régulée, au moyen du conduit 24.

On notera que sur la figure 1, les relations logiques entre les différents composants du dispositif de stabilisation sont symbolisées par des flèches. Elles sont présentes entre le système de contrôle-commande 3 et d'une part, l'appareil de test 2 et d'autre part, le dispositif de traitement 4.

A titre d'exemple, le Tableau 1 (voir fin de description) regroupe les principaux paramètres analytiques d'un vin traité par électrodialyse sur un pilote de laboratoire de surface membranaire de 0,1 m², à différents taux de désionisation de 0 % à 40 %. Le taux de désionisation nécessaire et suffisant pour obtenir la stabilité tartrique de ce vin était de 17 %. Sa conductivité initiale était de 3 105 µS/cm à 20°C.

Les paramètres analytiques du vin traité à 17 % de désionisation montrent que pour cette intensité de traitement, l'évolution du pH et de l'Acidité Totale restent dans des limites acceptables. En effet, par rapport au vin non traité (taux de désionisation : 0 %), ces deux paramètres diminuent respectivement, de 0.09 unité et 0.25 g/l (H₂SO₄). L'Acidité Volatile n'évolue pratiquement pas. Par contre à partir de 35 % de désionisation, la

diminution du pH, de l'Acidité Totale et de l'Acidité Volatile sont respectivement de 0.18 unité, 0.55 g/l et 0.05 g/l exprimés en H₂SO₄. Ces diminutions restent contenues dans des limites apparaissant acceptables au plan oenologique. Cependant, elles ne sont pas nécessaires et sont donc à éviter.

Parmi tous les vins rouges traités par le demandeur, il apparaît que la chute de conductivité à appliquer aux vins varie en général entre 5 % à 20 %. Elle est de 15 à 20 % pour les vins jeunes et de 5 à 15 % pour les autres vins.

A titre d'exemple, les tableaux 2 à 4 (voir fin de description), donnent l'ensemble des résultats analytiques obtenus lors d'un essai comparatif entre le procédé traditionnel par réfrigération et le procédé membranaire d'électrodialyse contrôlé.

Le vin témoin n'était pas stable. Le vin traité par électrodialyse était stable vis à vis des différents tests oenologiques usuels effectués après le traitement.

Pour les vins blancs et les vins types primeurs, ces pourcentages de chute de conductivité peuvent atteindre 30 %. L'ensemble de ces observations, confronté aux suivis analytiques correspondants, conduit à proposer une limite maximum de traitement à 30 % de désionisation. Le système de contrôle-commande permet d'assurer automatiquement le respect de toute limite maximum de traitement.

Ainsi, le système de contrôle-commande 3 gère automatiquement toutes les taches du procédé exécutées par le dispositif de stabilisation selon l'invention (remplissage des cuves, traitement du vin, vidange, rinçage, gestion des sécurités de fonctionnement) en agissant sur les dispositifs suivants : les électrovannes (8, 13, 15, 19), les pompes (11, 18, 20) et les régulateurs.

Le système de contrôle-commande gère également l'acquisition des informations et des données analogiques ou logiques pour :
- le contrôle du procédé,
- la régulation,
- le suivi événementiel du procédé.

On peut noter que pratiquement, le système de contrôle-commande est supervisé par une console interface (écran/clavier/périphériques) permettant d'assurer l'encadrement du procédé (contrôle, suivi et enregistrement des opérations).

Nous allons maintenant décrire plus en détail le procédé de traitement par électrodialyse lui-même, ainsi que l'électrodialyseur 10 qui permet sa mise en oeuvre.

L'électrodialyse est une méthode de séparation qui s'applique à des solutions ioniques. Elle met an jeu d'une part, un champ électrique qui constitue la force motrice du transport des ions en solution et d'autre part, des membranes perméables aux ions qui assurent la sélectivité du transport ionique et permettent de séparer les ions.

Les membranes cationiques ne laissent passer que les cations tandis que les membranes anioniques ne sont traversées que par les anions.

La figure 3 illustre le principe de fonctionnement d'une cellule d'électrodialyse qui constitue un motif élémentaire d'un électrodialyseur. Cette cellule est constituée de deux compartiments 31 et 32 qui sont délimités alternativement par des membranes anioniques 33 et 34 et cationique 35.

Une différence de potentiel appliquée aux bornes des électrodes (anode 36 et cathode 37) va entraîner la migration des ions. Les cations 38 vont se diriger vers la cathode 37 tandis que les anions 39 se déplacent vers l'anode 36. Les cations du compartiment 31 vont pouvoir traverser la membrane cationique 35 et être ainsi éliminés du compartiment 31. Cependant, ils ne peuvent pas sortir du compartiment 32 car ils trouvent sur leur chemin une membrane anionique, la membrane 34. De même, les anions 39 peuvent sortir du compartiment 31, par la membrane anionique 33, mais ils demeureront dans le compartiment 32 de la cellule d'électrodialyse car ils ne pourront pas franchir la membrane cationique 35.

Du fait de l'alternance des membranes anioniques et cationiques, l'un des compartiments de la cellule d'électrodialyse exporte ses ions vers les circuits voisins. Sa teneur en ions diminue et c'est pourquoi ce compartiment, ici 31, est appelé "diluat". Le compartiment 32 s'enrichit en ions et il est communément dénommé "concentrat".

Il convient de noter que la séparation des ions s'effectue sans réaction aux électrodes.

Un électrodialyseur est constitué par un empilement de cellules d'électrodialyse élémentaires. Entre chaque membrane, un cadre séparateur permet de maintenir entre les membranes une distance uniforme. Ce cadre assure également l'écoulement des fluides ainsi que la rigidité et l'étanchéité de l'empilement.

Un électrodialyseur peut comprendre jusqu'à 500 paires de membranes. Celles-ci sont assemblées selon une technologie proche de celle des filtre-presses. L'épaisseur des compartiments varie de 0,3 à 0,7 mm selon les fabrications. De façon classique, un électrodialyseur comprend deux circuits hydrauliques qui alimentent en parallèle l'ensemble des compartiments du même type (diluats et condensats).

Il est également équipé de deux électrodes (une anode et une cathode) qui sont placées & chacune des extrémités de l'empilement. Elles sont isolées dans un compartiment où circule une solution saline qui assure la conduction électrique. Ce compartiment n'a aucun contact avec le produit à traiter.

Les électrodes fournissent le courant d'alimentation de l'électrodialyseur. Elles sont alimentées par un courant électrique en continu. La différence de potentiel électrique, en général de l'ordre de 1 volt par cellule, qui est maintenue de part et d'autre de chaque membrane permet le passage sélectif des ions.

On constate que le rendement énergétique est d'environ 90 %.

Le procédé d'électrodialyse et le dispositif permettant sa mise en oeuvre sont connus dans l'état de la technique.

Pour pouvoir utiliser le procédé d'électrodialyse dans le cadre d'un procédé de stabilisation des vins, il est cependant nécessaire de choisir des membranes spécifiquement adaptées aux vins. En effet, l'électrodialyse ne doit modifier que la charge ionique des vins traités, tout en respectant l'équilibre entre les différentes espèces ioniques.

Ainsi, les membranes sélectionnées doivent posséder de bons flux vis à vis des espèces ioniques à extraire, c'est-à-dire les cations potassium et anions tartrates. Elles ne doivent pas affecter les constituants non ioniques du vin, en particulier les polyphénols et les polysacharides. Elles ne doivent pas non plus permettre la diffusion de petites molécules telles que l'éthanol. Pratiquement, la diminution maximale du degré alcoolique est fixée à 0,1 % vol. Enfin, elles doivent être résistantes au colmatage et au nettoyage régulier.

Il convient de rechercher le meilleur couple de membranes, cationique et anionique, de façon à ce que l'équilibre physico-chimique du vin soit peu modifié.

En effet, si l'on privilégie l'élimination du potassium des vins, on abaisse le pH. C'est ce qui se produit lorsqu'on utilise des résines échangeuses de cations. Si au contraire, on favorise l'extraction des tartrates, les autres anions (dont l'acétate) sont également exportés, ce qui entraîne une diminution de l'acidité volatile des vins.

Il faut de plus pouvoir contrôler et limiter le niveau de traitement. Les limites suivantes ont été fixées :
- diminution du pH inférieure à 0,2 unité,
- diminution de l'acidité volatile inférieure à 0, 09 g/l (exprimée en teneur de H₂SO₄).

On peut noter que la modification maximale du pH qui a été fixée est nettement inférieure à celle obtenue par les traitements par le froid (addition d'acide tartrique puis passage au froid) dans les conditions prévues par la réglementation.

Ces limites permettent d'effectuer un choix parmi les membranes disponibles sur le marché.

L'utilisation du procédé d'électrodialyse pour traiter les vins, en vue de leur stabilisation, comporte de nombreux avantages.

Tout d'abord, de nombreux contrôles ont été effectués en vue de déterminer la stabilité tartrique de vins traités par le froid et par le procédé selon l'invention.

Les tests de stabilité utilisés sont classiques test de la tenue au froid (stabilisation en tubes à essais pendant 15 jours à - 4°C) et de la mesure de la température de saturation.

Ces tests ont mis en évidence l'efficacité du procédé selon l'invention, aussi bien sur des vins jeunes que sur des vins d'un an ou plus.

En revanche, la stabilité n'a pas toujours été obtenue par les différents traitements au froid (discontinus par stabilisation ou continus avec ensemencement).

Des contrôles analytiques ont également été effectués sur la base des paramètres suivants :
- densité,
- sucres totaux,
- titre alcoométrique volumique,
- méthanol,
- glycérol,
- alcools supérieurs,
- éthanal,
- azote total, ammoniacal, acides aminés
- acidité totale, pH, acidité volatile,
- acides organiques tartrique, malique, lactique,
- SO₂ libre et total,
- K⁺, Na⁺, Ca⁺⁺,
- Fe, Cu, Mg, Mn,
- absorbances à 520, 420 et 280 nm,
- polyphénols totaux,
- état de la matière colorante,
- profil polysaccharidique des vins.

Les nombreuses analyses réalisées mettent clairement en évidence que seuls les cations et les anions du vin sont concernés par le traitement d'électrodialyse et que par conséquent, les autres constituants du vin, comme l'éthanol, les composés volatils, les polyphénols et les acides aminés, ne sont pas ou très peu affectés par ce traitement.

De plus, il a été vérifié que le procédé selon l'invention entraîne des modifications moins importantes de la matière colorante que les traitements classiques de stabilisation par le froid.

En effet, un traitement par le froid provoque non seulement la précipitation de bitartrate de potassium mais aussi la précipitation de matière colorante.

Au contraire, du fait de l'absence de pores des membranes d'électrodialyse (membranes denses), seuls les ions de faible encombrement stériques sont exportés. Ainsi l'électrodialyse préserve mieux la composante colloïdale des vins par rapport & un traitement par réfrigération. Des différences sensibles ont pu être notées au niveau de la matière colorante colloïdale des vins rouges.

Par conséquent, sur certains vins, une différence significative peut apparaître, au travers d'une dégustation triangulaire entre le vin traité au froid et le vin traité par le procédé selon l'invention.

Par contre il n'apparaît pas de différences significative entre les vins, avant et après le traitement selon le procédé conforme à l'invention.

En effet, la mise en oeuvre du procédé d'électrodialyse est très favorable à la préservation des qualités organoleptiques du vin, car toutes les conditions de transfert sont très douces :
- le vin circule en film mince dans les compartiments et à l'abri de tout contact avec l'air,
- la vitesse de passage du vin sur les membranes est relativement faible, de l'ordre de 6 cm par seconde,
- l'écoulement s'effectue à basse pression (inférieure à 1 bar), à température contrôlée (20°C à 25°C) et sous protection de gaz inerte,
- le temps moyen de séjour du vin au contact des membranes est relativement court, de l'ordre de 30 secondes.

Le vin n'est soumis à aucun effet électrochimique car les électrodes sont isolées dans un compartiment où ne circule qu'une solution de sel de potassium.

De plus, contrairement aux techniques mettant en oeuvre les échangeurs d'ions, une membrane d'électrodialyse n'a pas besoin d'être régénérée. Après chaque cycle de travail, les membranes sont nettoyées avec des solutions faiblement alcalines ou acides, comme pour toute technique membranaire.

Le procédé de stabilisation comporte des avantages importants par rapport aux traitements classiques par le froid puisqu'il peut s'adapter à l'équilibre physico-chimique de chaque vin à traiter, en éliminant uniquement la quantité de potassium et d'acide tartrique nécessaire à l'obtention de la stabilité.

Le système de contrôle-commande permet de conduire le procédé de traitement d'une part, en empêchant le traitement d'un vin stable et d'autre part, en fixant un niveau de traitement maximum (par exemple un taux de désionisation maximum). Ceci permet d' interdire un usage excessif et inutile de l'électrodialyse, entraînant en particulier une diminution du pH ou de l'acidité volatile du vin traité. Il permet également d'empêcher le traitement en cas de substitution de vins.

De plus, le procédé de traitement par électrodialyse comporte moins d'étapes qu'un procédé de traitement par refrigération. Les filtrations qui sont nécessaires en aval et en amont du traitement de réfrigération se réduisent à une simple préfiltration, pour un traitement par électrodialyse. Il en résulte des économies de produits consommables, tels que les adjuvants de filtration, les tartres d'ensemencement et l'acide métatartrique.

L'électrodialyse permet également de réduire les dépenses en énergie. En effet, il a été évalué que la dépense énergétique liée au traitement par réfrigération est au moins dix fois supérieure & celle liée au procédé de traitement par électrodialyse.

L'automatisation du procédé d'électrodialyse permet de réduire le coût de sa mise en oeuvre. Il a été évalué que, selon les cas, le coût de la main d'oeuvre intervenant dans un procédé de stabilisation tartrique des vins peut être réduit d'un facteur de 2 à 8 avec le procédé de traitement selon l invention.

Enfin, le concentrat d'électrodialyse est un effluant liquide, peu polluant, riche en acide tartrique, qui est valorisable.

Ainsi, le procédé et le dispositif de stabilisation tartrique des vins conformes à l'invention permettent de résoudre les problèmes de précipitations tartriques spécifiques à chaque vin, de façon rationnelle et contrôlée.

L'appareil de test 2 a été décrit comme un élément du dispositif de traitement selon l'invention. On peut noter que cet appareil peut être utilisé indépendamment du dispositif de traitement, en tant qu'appareil de mesure destiné à apprécier le degré de stabilité d'un échantillon de vin. Dans ce cas, l'appareil de test peut ne mettre en oeuvre que les deux premières étapes du procédé décrit précédemment, puisqu'il n'est pas nécessaire de déterminer une valeur de consigne.

L'appareil peut également être utilisé dans le cadre de procédé de traitement ne mettant pas en oeuvre la technique de l'électrodialyse.

Les signes de référence insérés après les caractéristiques techniques mentionnées dans les revendications, ont pour seul but de faciliter la compréhension de ces dernières, et n'en limitent aucunement la portée.

**TABLEAU 1**

| EVOLUTION DES PARAMETRES ANALYTIQUES D'UN VIN EN FONCTION DU TAUX DE DESIONISATION (Essai pilote laboratoire) VIN ROUGE 1992 | | | | | | | | | |
|---|---|---|---|---|---|---|---|---|---|
| Taux de désionisation | | 0 % | 10 % | 17 % | 20 % | 25 % | 30 % | 35 % | 40 % |
| TAV % Vol. à 20°C (Titre alcoométrique volumique) | | 10.60 | 10.60 | 10.60 | 10.60 | 10.55 | 10.50 | 10.40 | 10.35 |
| AT (H₂SO₄) (Acidité totale) | g/l | 3.10 | 3.00 | 2.85 | 2.80 | 2.75 | 2.65 | 2.55 | 2.50 |
| pH | | 3.84 | 3.79 | 3.75 | 3.74 | 3.72 | 3.71 | 3.66 | 3.64 |
| AT H₂SO₄ (Acidite volatile) | g/l | 0.55 | 0.53 | 0.54 | 0.54 | 0.54 | 0.53 | 0.50 | 0.52 |
| Ac. Tartrique | g/l | 2.60 | 2.20 | 1.80 | 1.80 | 1.60 | 1.40 | 1.20 | 1.00 |
| Ac. Lactique | g/l | 1.40 | 1.40 | 1.40 | 1.40 | 1.40 | 1.40 | 1.30 | 1.30 |
| SO₂ Libre | mg/l | 8 | 8 | 8 | 9 | 9 | 10 | 8 | 8 |
| SO₂ Total | mg/l | 32 | 29 | 31 | 30 | 26 | 31 | 31 | 29 |
| K⁺ | mg/l | 1 690 | 1 440 | 1 280 | 1 190 | 1 100 | 990 | 860 | 780 |
| Ca⁺⁺ | mg/l | 60 | 69 | 67 | 67 | 60 | 67 | 67 | 64 |
| Na⁺ | mg/l | 21.7 | 20.0 | 18.9 | 18.5 | 17.9 | 16.9 | 15.6 | 14.7 |
| DO 280 nm | | 40.7 | 39.7 | 39.4 | 39.5 | 38.9 | 38.5 | 37.5 | 37.5 |

**TABLEAU 2**

| RESULTATS D'ANALYSE D'ESSAIS COMPARATIFS ENTRE LE PROCEDE TRADITIONNEL PAR REFRIGERATION ET LE PROCEDE MEMBRANAIRE D'ELECTRODIALYSE CONTROLE | | | |
|---|---|---|---|
| Analyses physico-chimiques | | | |

| BORDEAUX ROUGE 1988 | TO | TF | ED |
|---|---|---|---|
| Densité | 0,9921 | 0,9921 | 0,9920 |

| Sucres : | | | |
|---|---|---|---|
| -Glucose g/l | 0,55 | 0,55 | 0,55 |
| -Fructose g/l | 0,25 | 0,25 | 0,20 |
| Alcool % vol. | 11°85 | 11°85 | 11°81 |
| Ac. Totale H2SO4 g/l | 3,45 | 3,40 | 3,40 |
| pH | 3,58 | 3,57 | 3,51 |
| Ac. Volatile H2SO4 g/l | 0,49 | 0,49 | 0,43 |
| Ac. Tartrique g/l | 2,3 | 2,4 | 2,3 |
| Ac. Malique | 0 | 0 | 0 |
| Ac. Lactique | 1,90 | 1,90 | 1,85 |
| SO2 Libre | 22 | 16 | 21 |
| SO2 Total | 80 | 70 | 80 |
| K+ mg/l 1160 | | 1130 | 1090 |
| Na+ mg/l | 18 | 18 | 18 |
| Ca++ mg/l | 76 | 76 | 66 |
| Fe mg/l | 6,8 | 6,6 | 6,5 |
| Plomb µg/l | 62 | 58,5 | 55 |
| Conductivité µs à 20°C | 2135 | 2100 | 1980 |

| | | | |
|---|---|---|---|
| TO = Témoin(vin filtré) | | | |
| TF = Traité par le froid (8 jours à -2°C) | | | |
| ED = Traité par électrodialyse | | | |

**TABLEAU 3**

| RESULTATS D'ANALYSE D'ESSAIS COMPARATIFS ENTRE LE PROCEDE TRADITIONNEL PAR REFRIGERATION ET LE PROCEDE MEMBRANAIRE D' ELECTRODIALYSE CONTROLE | | | |
|---|---|---|---|
| Analyses des polyphénols, de la couleur et des polysaccharides | | | |

| | TO | TF | ED |
|---|---|---|---|
| Composition en Anthocyanes: | | | |
| -Anthocyenes totales | 332 | 304 | 326 |
| -Indice PVP | 44 | 43 | 44 |
| -Indice d'ionisation | 18 | 21 | 17 |
| - Antho. libres mg/l | 185 | 174 | 183 |
| - Antho. combinées aux tanins | 147 | 130 | 143 |
| -Anthoc. colorées | 59,8 | 63,8 | 55,4 |
| -Indice de chauffage | 22 | 22 | 20 |
| -Indice de pigments polymérisés | 56 | 55 | 57 |

| Composition en tanins: | | | |
|---|---|---|---|
| -Procyanidins mg/l | 2816 | 2763 | 2807 |
| -DO 280 (x dil) | 45 | 43,2 | 44,5 |
| -Folin Ciocaiteur (DO 700 x dil x 100) | 225 | 218 | 224 |
| -Procyanidins/Anthoc. | 8,5 | 9,1 | 8,6 |
| -Tanins précipités par HCl % | 21 | 22 | 24 |
| -Tanins précipités par gélatine % | 59 | 61 | 60 |
| -Tanins précipités par éthanol % | 17 | 10 | 13 |
| -Tanins g/l | 3,60 | 3,46 | 3,56 |

| Coloration: | | | |
|---|---|---|---|
| -Intensité colorante DO420+DO520+DO620-10mm | 6,15 | 6,07 | 6,26 |
| -Teinte DO420/DO520 | 0,77 | 0,74 | 0,76 |
| DO 420 % | 38,70 | 38,05 | 38,50 |
| DO 520 % | 51,40 | 50,40 | 50,80 |
| DO 620 % | 10,90 | 10,55 | 10,70 |

| Origine de la couleur rouge: | | | |
|---|---|---|---|
| -DO 520 (vin) 1 mm | 0,310 | 0,312 | 0,318 |
| -DO 520 (Al) 1mm % | 0,055 | 0,057 | 0,065 |
| -DO 520 (TA) 1 mm % | 0,149 | 0,161 | 0,130 |
| -DO 520 (TAT) 1 mm % | 0,106 | 0,094 | 0,122 |
| -DO 520 (TA)/TA g/l | 1,01 | 1,24 | 0,91 |
| -dialyse % | 19 | 17 | 19 |

| Autres polymères: | | | |
|---|---|---|---|
| -Colloïdes décolorés mg/l | 686 | 672 | 702 |
| -Polysaccharides neutres mg/l | 284 | 234 | 283 |
| -Polysaccharides acides mg/l | 87 | 75 | 96 |

**TABLEAU 4**

| RESULTATS D'ANALYSE D'ESSAIS COMPARATIFS ENTRE LE PROCEDE TRADITIONNEL PAR REFRIGERATION ET LE PROCEDE MEMBRANAIRE D'ELECTRODIALYSE CONTROLE | | | |
|---|---|---|---|
| Analyses des acides aminés et de composés volatils | | | |

| | TO | TF | ED |
|---|---|---|---|
| Composés azotés: | | | |
| en n.moles/50 µl vin | | | |
| -Acide Aspartique | 2,14 | 2,16 | 2,69 |
| -Thréomine | 0,88 | 0,97 | 0,98 |
| -Serine | 1,60 | 1,62 | 1,58 |
| -Acide Glutamique | 6,69 | 7,36 | 6,92 |
| -Proline | 376,6 | 365,9 | 370,7 |
| - Glycocol | 7,33 | 7,90 | 8,36 |
| -Alamine | 15,03 | 17,16 | 17,12 |
| -Valine | 3,98 | 4,99 | 2,11 |
| -Cystine | - | - | - |
| -Methionine | 0,99 | 0,92 | 0,90 |
| -Isoleucine | 1,30 | 1,37 | 1,48 |
| -Leucine | 2,63 | 2,85 | 3,14 |
| -Tyrosine | 1,24 | 1,34 | 1,42 |
| -Phenylalanine | 2,30 | 2,51 | 2,60 |
| -Acide amino butyrique | 2,68 | 3,10 | 3,98 |
| -Ethanolamine | 9,86 | 11,87 | 11,07 |
| -Ornithine | 3,03 | 2,65 | 2,76 |
| -Lysine | 3,70 | 3,99 | 4,24 |
| -Histidine | 1,84 | 1,99 | 1,39 |
| -Arginine | 3,71 | 2,39 | 4,45 |
| -NH3 | 14,73 | 16,18 | 22,05 |

| Composés volatils: | | | |
|---|---|---|---|
| -Ethanol (*) | 11°85 | 11°85 | 11°81 |
| -Ethanai (*) | 23,60 | 22,05 | 25,60 |
| -Acétate d'éthyle (*) | 52,90 | 57,20 | 50,85 |
| -Propanol (*) | 16,25 | 16,05 | 15,45 |
| -Isobutanol (*) | 79,85 | 76,15 | 76,50 |
| -Isopentanols (*) | 331,70 | 325,70 | 324,75 |
| -Méthanol mg/l | 154 | 145 | 149 |

| | | | |
|---|---|---|---|
| (*) en g/hl/Al. Pur | | | |

## Revendications

1. Procédé régulé de stabilisation tartrique des vins comprenant les étapes suivantes :
- prélèvement d'un échantillon du vin à stabiliser;
- détermination du degré d'instabilité spécifique dudit vin, par une mesure prédictive de conductivité correspondant à celle qu'aurait le vin s'il était stable ;
- si le vin est instable, traitement dudit vin à stabiliser selon une technique membranaire d'électrodialyse jusqu'à l'obtention de son état de stabilité correspondant à une valeur de consigne, la mesure prédictive de conductivité étant transformée en valeur de consigne correspondant à la chute de conductivité nécessaire à la stabilisation du vin à traiter.

2. Procédé conforme à la revendication 1, selon lequel le vin à stabiliser est soumis en continu à un traitement par électrodialyse.

3. Procédé conforme à l'une des revendications 1 ou 2, selon lequel la mesure prédictive de conductivité est obtenue à partir d'un calcul de modélisàtion.

4. Procédé conforme à l'une des revendications 1 à 3, selon lequel le traitement est effectué sur la totalité du vin à stabiliser, en un seul cycle, de façon continue.

5. Procédé conforme à l'une des revendications 1 à 3, selon lequel le traitement est effectué de façon continue, par cycles successifs, un volume intermédiaire étant traité à chaque cycle, jusqu'au traitement de la totalité du vin à stabiliser.

6. Procédé conforme à l'une des revendications 1 à 5, selon lequel le vin à stabiliser est filtré avant le traitement par électrodialyse.

7. Procédé conforme à l'une des revendications 1 à 6, selon lequel le traitement est limité à un taux maximum de désionisation préalablement déterminé, par exemple d'environ 30%.

8. Procédé conforme à l'une des revendications 1 à 7, selon lequel la concentration en ions du concentré résultant du traitement par électrodialyse est maintenue sous un seuil déterminé.

9. Procédé conforme à la revendication 8, selon lequel le maintien sous ledit seuil est assuré par la régulation de la conductivité du concentré par apport d'eau.

10. Dispositif automatique de stabilisation tartrique des vins comprenant:
- un appareil de test (2) pour déterminer le degré d'instabilité spécifique du vin à stabiliser, par une mesure prédictive de conductivité correspondant à la conductivité qu'aurait le vin s'il était stable;
- un dispositif de traitement (4) comprenant un électrodialyseur (10) et recevant le vin à stabiliser;
- un système de contrôle-commande (3) pour gérer automatiquement le dispositif de traitement (4), en fonction du degré d'instabilité du vin à traiter, déterminé par l'appareil de test (2).

11. Dispositif conforme à la revendication 10, selon lequel la mesure prédictive de conductivité est transformée en une valeur de consigne correspondant à la chute de conductivité nécessaire à la stabilisation du vin à traiter.

12. Dispositif conforme à la revendication 11, selon lequel le dispositif de traitement (4) comprend en outre un dispositif de mesure (21) pour mesurer la conductivité du vin en sortie de l'électrodialyseur (10), le système de contrôle-commande (3) assurant le retour en continu du vin à stabiliser dans l'électrodialyseur (10) par des moyens appropriés (15, 14, 6, 9, 11), si la valeur de consigne n'est pas atteinte.

13. Dispositif conforme à la revendication 12, selon lequel le système de contrôle-commande (3) assure l'évacuation du vin du dispositif de traitement (4) grâce à des moyens appropriés (12, 13), lorsque la valeur de consigne est atteinte.

14. Dispositif conforme aux revendications 10 à 13, selon lequel il est utilisé entre une première cuve (1) comprenant la totalité du vin à stabiliser et une deuxième cuve (5) qui recueille le vin stabilisé.

15. Dispositif conforme à l'une des revendications 10 à 14, selon lequel le dispositif de traitement (4) est directement relié à ladite première cuve (1) contenant la totalité du vin à stabiliser.

16. Dispositif conforme à l'une des revendications 10 à 14, selon lequel le dispositif de traitement (4) comprend une cuve intermédiaire (6) associée à l'électrodialyseur (10) et recevant un volume de vin à stabiliser.

17. Dispositif conforme à la revendication 16 selon lequel, lorsque le volume de vin présent dans la cuve intermédiaire (6) au début du traitement a été évacué, le système de contrôle (3) agit sur des moyens appropriés (8, 20) pour assurer son remplissage par un nouveau volume de vin à stabiliser.

18. Dispositif conforme à la revendication 17 caractérisé en ce que la vidange et le remplissage de la cuve intermédiaire (6) s'effectuent de telle sorte que le vin est traité de façon continue dans l'électrodialyseur (10).

19. Dispositif conforme aux revendications 10 à 18, selon lequel le dispositif de traitement (4) comprend également un filtre (22) placé en amont de l'électrodialyseur (10).

20. Dispositif conforme aux revendications 10 à 19, selon lequel le dispositif de traitement (4) inclut en outre un circuit comprenant une boucle (16) reliant l'électrodialyseur (10) à une cuve (17) recevant le concentré d'ions.

21. Dispositif conforme à la revendication 20, selon lequel le dispositif de traitement (4) comprend des moyens (23, 19) pour réguler le seuil de conductivité dudit concentré.

22. Dispositif conforme aux revendications 10 à 21, selon lequel l'appareil de test (2) réalise une fiche d'identité du vin.

23. Dispositif conforme à la revendication 22, selon lequel il comprend un dispositif de sécurité pour interdire le traitement du vin dans l'électrodialyseur (10) lorsque les caractéristiques de celui-ci ne sont pas conformes à sa fiche d'identité.

## Claims

1. Regulated method for the tartaric stabilization of wines comprising the following steps:
- taking a sample of the wine to be stabilized,
- determining the specific instability degree of said wine, from a predicted conductivity value corresponding to that which the wine would have if it were stable,
- if the wine is unstable, said wine to be stabilized is treated according to a membrane electrodialysis technique until it becomes stable corresponding to a reference value, whereas the predicted conductivity value is converted into a reference value corresponding to the decrease in conductivity necessary for the stabilization of the wine to be treated.

2. Method according to claim 1, wherein the wine to be stabilized is subjected continously to an electrodialysis treatment.

3. Method according to one of claims 1 or 2, wherein the predicted conductivity value is obtained from a model calculation.

4. Method according to one of claims 1 to 3, wherein the treatment is carried out on the whole of the wine to be stabilized, continuously, in a single cycle.

5. Method according to one of claims 1 to 3, wherein the treatment is carried out continuously, by successive cycles, an intermediate volume being treated in each cycle, until the whole of the wine to be stabilized has been treated.

6. Method according to one of claims 1 to 5, wherein the wine to be stabilized is filtered before the electrodialysis treatment.

7. Method according to one of claims 1 to 6, wherein the treatment is limited to a previously determinated maximum deionization level, for exemple about 30 %.

8. Method according to one of claims 1 to 7, wherein the ionic concentration of the concentrate resulting from the electrodialysis treatment is maintened below a fixed threshold.

9. Method according to claim 8, wherein the maintenance below said threshold is achieved by regulation of the conductivity of the concentrate by addition of water.

10. Automatic device for the tartaric stabilization of wines comprising:
- a test apparatus (2) to determine the specific instability degree of the wine to be stabilized, from a predicted conductivity value corresponding to the conductivity which the wine would have if it were stable,
- a treatment device (4) comprising an electrodialyser (10) and receiving the wine to be stabilized,
- a control system (3) to control automatically the treatment device (4), as a function of the instability degree of the wine to be treated, determined by the test apparatus (2).

11. Device according to claim 10, wherein the predicted conductivity value is converted into a reference value corresponding to the decrease in conductivity necessary for the stabilization of the wine to be treated.

12. Device according to claim 11, wherein the treatment device (4) moreover comprises a measuring device (21) for measuring the conductivity of the wine on leaving the electrodialyzer (10), the control system (3) ensuring the continuous return of the wine to be stabilized into the electrodialyzer (10) by suitable means (15, 14, 6, 9, 11), if the reference value is not yet attained.

13. Device according to claim 12, wherein the control system (3) ensures the evacuation of the wine from the treatment device (4) using suitable means (12, 13), when the reference value is attained.

14. Device according to claims 10 to 13, wherein it is used between a first vat (1) containing the whole of the wine to be stabilized and a second vat (5) which collects the stabilized wine.

15. Device according to one of claims 10 to 14, wherein the treatment device (4) is directly linked to said first vat (1) containing the whole of the wine to be stabilized.

16. Device according to one of claims 10 to 14, wherein the treatment device (4) comprises an intermediate vat (6) linked to the electrodialyzer (10) and receiving a volume of wine to be stabilized.

17. Device according to claim 16, wherein when the volume of wine present in the intermediate var (6) at the beginning of the treatment has been evacuated, the control system (3) acts on suitable means (8, 20) to ensure its filling by a new volume of wine to be stabilized.

18. Device according to claim 17, characterized in that the emptying and filling of the intermediate vat (6) are carried out in such a way that the wine is treated continuously in the electrodialyzer (10).

19. Device according to claims 10 to 18, wherein the treatment device (4) also comprises a filter (22) situated upstream of the electrodialyzer (10).

20. Device according to claims 10 to 19, wherein the treatment device (4) moreover comprises a circuit comprising a loop (16) linking the electrodialyzer (10) to a vat (17) receiving the ion concentrate.

21. Device according to claim 20, wherein the treatment device (4) comprises means (23, 19) to regulate the conductivity threshold of said concentrate.

22. Device according to claims 10 to 21, wherein the test apparatus (2) produces an identification record for the wine.

23. Device according to claim 22, wherein it comprises a safety device to prevent the treatment of the wine in the electrodialyzer (10) when the characteristics of the wine do not conform to its identification record.

## Patentansprüche

1. Geregeltes Verfahren zur Tartrat-Stabilisierung von Weinen, umfassend die folgenden Stufen:
- Entnahme einer Probe des zu stabilisierenden Weins,
- Bestimmung des spezifischen Instabilitätsgrades des Weins durch eine Vorgabemessung des Leitfähigkeitswerts, den der Wein hätte, wenn er stabil wäre,
- wenn der Wein instabil ist, die Behandlung des zu stabilisierenden Weins gemäß einer Membran-Elektrodialyse-Technik bis zur Erzielung seines einem vorgegebenen Leitfähigkeitswert entsprechenden Stabilitätszustands, wobei die Vorgabemessung in eine Wertvorgabe für den zur Stabilisierung des zu behandelnden Weins erforderlichen Leitfähigkeitswertsabfall umgewandelt wird.

2. Verfahren nach Anspruch 1, wobei der zu stabilisierende Wein einer kontinuierlichen Elektrodialyse-Behandlung unterzogen wird.

3. Verfahren nach Anspruch 1 oder 2, wobei die Wertvorgabe des Leitfähigkeitswerts aus einer Modellrechnung erhalten wird.

4. Verfahren nach einem der Ansprüche 1 bis 3, wobei die Behandlung in einem einzigen Zyklus auf kontinuierliche Weise auf die Gesamtmenge des zu stabilisierenden Weins angewandt wird.

5. Verfahren nach einem der Ansprüche 1 bis 3, wobei die Behandlung auf kontinuierliche Weise durch aufeinanderfolgende Zyklen durchgeführt wird, wobei in jedem Zyklus ein Zwischenvolumen behandelt wird, bis die Gesamtmenge des zu stabilisierenden Weins behandelt ist.

6. Verfahren nach einem der Ansprüche 1 bis 5, wobei der zu stabilisierende Wein vor der Elektrodialyse-Behandlung filtriert wird.

7. Verfahren nach einem der Ansprüche 1 bis 6, wobei die Behandlung auf einen zuvor bestimmten, maximalen Deionisationsgrad, zum Beispiel etwa 30 %, begrenzt wird.

8. Verfahren nach einem der Ansprüche 1 bis 7, wobei die Ionenkonzentration des aus der Elektrodialyse-Behandlung resultierenden Konzentrats unter einem bestimmten Schwellenwert gehalten wird.

9. Verfahren nach Anspruch 8, wobei die Aufrechterhaltung des Schwellenwerts durch die Regelung der Leitfähigkeit des Konzentrats durch Zugabe von Wasser sichergestellt wird.

10. Automatische Vorrichtung zur Tartrat-Stabilisierung von Weinen, umfassend:
- ein Prüfgerät (2) zur Bestimmung des spezifischen Instabilitätsgrads des zu stabilisierenden Weins durch eine Vorgabemessung des Leitfähigkeitswerts, den der Wein hätte, wenn er stabil wäre,
- eine Behandlungsvorrichtung (4), umfassend einen Elektrodialiseur (10), der den zu stabilisierenden Wein aufnimmt,
- ein Kontroll-Steuersystem (3) zur automatischen Führung der Behandlungsvorrichtung (4) als Funktion des durch das Prüfgerät (2) bestimmten Instabilitätsgrads des zu behandelnden Weins.

11. Vorrichtung nach Anspruch 10, wobei die Vorgabemessung in eine Wertvorgabe für den zur Stabilisierung des zu behandelnden Weins erforderlichen Leitfähigkeitswertsabfall umgewandelt wird.

12. Vorrichtung nach Anspruch 11, wobei die Behandlungsvorrichtung (4) außerdem eine Meßvorrichtung (21) zur Messung der Leitfähigkeit des aus dem Elektrodialiseur (10) austretenden Weins umfaßt, wobei das Kontroll-Steuersystem (3) die kontinuierliche Rückführung des zu stabilisierenden Weins durch geeignete Mittel (15, 14, 6, 9, 11) in den Elektrodialiseur (10) sicherstellt, wenn der vorgegebene Wert nicht erreicht ist.

13. Vorrichtung nach Anspruch 12, wobei das KontrollSteuersystem (3) beim Erreichen des vorgegebenen Werts mit Hilfe geeigneter Mittel (12, 13) die Abführung des Weins aus der Behandlungsvorrichtung (4) sicherstellt.

14. Vorrichtung nach Anspruch 10 bis 13, wobei die Vorrichtung zwischen einem ersten Behälter (1), der die Gesamtmenge des zu stabilisierenden Weins enthält, und einem zweiten Behälter (5), der den stabilisierten Wein aufnimmt, verwendet wird.

15. Vorrichtung nach einem der Ansprüche 10 bis 14, wobei die Behandlungsvorrichtung (4) direkt mit dem ersten Behälter (1), der die Gesamtmenge des zu stabilisierenden Weins enthält, verbunden ist.

16. Vorrichtung nach einem der Ansprüche 10 bis 14, wobei die Behandlungsvorrichtung (4) einen mit dem Elektrodialiseur (10) verbundenen Zwischenbehälter (6) umfaßt, der ein Volumen des zu stabilisierenden Weins aufnimmt.

17. Vorrichtung nach Anspruch 16, wobei, wenn das in dem Zwischenbehälter (6) zu Beginn der Behandlung vorhandene Weinvolumen abgelassen wurde, das Kontrollsystem (3) so auf die geeigneten Mittel (8, 20) einwirkt, daß dessen Auffüllung durch ein neues Volumen des zu stabilisierenden Weins sichergestellt wird.

18. Vorrichtung nach Anspruch 17, dadurch gekennzeichnet, daß die Entleerung und die Befüllung des Zwischenbehälters (6) so bewerkstelligt werden, daß der Wein im Elektrodialiseur (10) auf kontinuierliche Weise behandelt wird.

19. Vorrichtung nach Anspruch 10 bis 18, wobei die Behandlungsvorrichtung (4) darüber hinaus einen Filter (22) umfaßt, der stromaufwärts vom Elektrodialiseur (10) angeordnet ist.

20. Vorrichtung nach Anspruch 10 bis 19, wobei die Behandlungsvorrichtung (4) außerdem einen Kreislauf enthält, der eine Schleife (16) umfaßt, die den Elektrodialiseur (10) mit einem Behälter (17) verbindet, der das Ionenkonzentrat aufnimmt.

21. Vorrichtung nach Anspruch 20, wobei die Behandlungsvorrichtung (4) Mittel (23, 19) zur Regelung des Leitfähigkeits-Schwellenwerts des Konzentrats enthält.

22. Vorrichtung nach Anspruch 10 bis 21, wobei das Prüfgerät (2) eine Datei mit Kennwerten des Weins erstellt.

23. Vorrichtung nach Anspruch 22, umfassend eine Sicherheitsvorrichtung, die die Behandlung des Weins im Elektrodialiseur (10) verhindert, wenn dessen Merkmale nicht denen seiner Kennwert-Datei entsprechen.
